# FASCICULE DE BREVET EUROPEEN

(11) **EP 1 075 268 B1**
(45) Date de publication et mention de la délivrance du brevet: **27.07.2005**
(21) Numéro de dépôt: 99914615.2
(22) Date de dépôt: 20.04.1999
(51) Int. Cl.: A61K 33/16, A61K 31/00, A61K 7/18, A61P 31/00

(54) **UTILISATION D'UN VECTEUR D'IONS FLUORURE POUR LA PREPARATION D'UN MEDICAMENT DESTINE A LA PREVENTION OU AU TRAITEMENT DE MALADIES ASSOCIEES A HELICOBACTER PYLORI**
VERWENDUNG VON FLUORIDIONEN ZUR HERSTELLUNG EINES ARZNEIMITTELS ZUR PRÄVENTION UND/ODER THERAPIE VON MIT HELICOBACTER PYLORI ZUSAMMENHÄNGENDEN ERKRANKUNGEN
USE OF A FLUORIDE ION VECTOR FOR PREPARING A MEDICINE FOR PREVENTING OR TREATING DISEASES ASSOCIATED WITH HELICOBACTER PYLORI

(30) Priorité: 04.05.1998 FR 9805597
(43) Date de publication de la demande: 14.02.2001
(73) Titulaire: Sanofi-Aventis, 75013 Paris (FR)
(72) Inventeur: FINIDORI, Claudine, F-92120 Montrouge (FR)
(74) Mandataire: Thouret-Lemaitre, Elisabeth
(86) Numéro de dépôt international: PCT/FR1999/000920
(87) Numéro de publication internationale: WO 1999/056756

(56) Documents cités:
- WO-A-96/24341
- NAKAO, M. ET AL: "Antibacterial properties of lansoprazole alone and in combination with antimicrobial agents against Helicobacter pylori" EUR. J. CLIN. MICROBIOL. INFECT. DIS., 1995, 14, 391-9, XP002094215

## Description

La présente invention a pour objet des améliorations dans la prévention et le traitement de maladies associées à Helicobacter pylori, en particulier les ulcères gastro-intestinaux.

Il est bien connu que Helicobacter pylori est une bactérie associée à certaines maladies gastro-intestinales, en particulier les gastrites et les ulcères digestifs, notamment les ulcères gastriques et les ulcères duédénaux, et qu'il représente un facteur de risque dans le cancer de l'estomac.

Plus récemment, il a été démontré qu'Helicobacter pylori était présent dans la plaque dentaire (Nguyen et al., Journal of Clinical Microbiology 31(4) 783-787, 1993) et pouvait être transmis oralement (L. Cellini et al., Microbiologica, 1995, 18: 187-92 et I.M Madinier et al., J. Periodontol, 1997 : 68 :2-6).

La demande internationale WO 96/24341 a proposé d'éradiquer Helicobacter pylori de la plaque dentaire en vue d'améliorer le traitement de l'ulcère digestif et de prévenir sa récidive, au moyen de formulations d'applications buccales comprenant des dérivés du bismuth. Cependant, ces dérivés ont pour inconvénient d'être toxiques et ne peuvent être administés sur une longue période de temps sans inconvénient pour les patients.

La demanderesse a pu alors mettre en évidence que, de manière surprenante, des vecteurs d'ions fluorure présentaient la propriété d'éradiquer ou de diminuer de manière significative la population d'Helicobacter pylori dans la cavité buccale.

La présente invention consiste en l'utilisation d'au moins un vecteur d'ions fluorure pharmaceutiquement acceptables pour la préparation d'un médicament destiné au traitement adjuvant ou la prévention d'une maladie associée à Helicobacter pylori.

Selon un autre aspect de l'invention, celle-ci consiste en l'utilisation d'au moins un vecteur d'ions fluorure pharmaceutiquement acceptables pour la préparation d'un médicament destiné à l'éradication d'Helicobacter pylori dans la cavité buccale.

Au sens de la présente invention, on entend par le terme "vecteur d'ions fluorure" un composé fluoré, minéral ou organique, qui autorise la libération d'ions fluorure dans un milieu aqueux tel la salive.

Un vecteur d'ions fluorure pharmaceutiquement acceptable peut être choisi dans le groupe constitué par :
(i) les fluorures d'amines ; le fluorure d'ammonium ; les fluorures de métaux alcalins, en particulier de sodium et de potassium ; les fluorures alcalino-terreux, en particulier de calcium ; les fluorures de titane, de zinc, d'aluminium, ainsi que les dérivés de ces fluorures ;
(ii) les fluorophosphates d'aluminium ; les fluorophosphates de métaux alcalins, en particulier de sodium et de potassium, plus particulièrement le monofluorophosphate de sodium ; les fluorophosphates alcalino-terreux, en particulier de calcium ; les fluorophosphates d'aluminium, en particulier le monofluorophosphate d'aluminium ;
(iii) le difluorophosphate d'aluminium ;
(iv) le fluorozirconate de sodium ;
(v) les fluorosilicates, tels que les fluorosilicates de sodium comme l'hexafluorosilicate de sodium.

Des vecteurs d'ions fluorure pharmaceutiquement acceptables particulièrement préférés consistent en le fluorure de sodium, le monofluorophosphate de sodium ou un mélange de fluorure de sodium et de monofluorophosphate de sodium.

Généralement, un médicament selon l'invention comprend des vecteurs d'ions fluorure pharmaceutiquement acceptables en une quantité telle que sa teneur en ions fluorure soit supérieure à 0,015 % en poids, de préférence comprise entre 0,15 et 1% en poids par rapport au poids total dudit médicament.

Un médicament conforme à l'invention se présente généralement sous une forme pour application buccale locale, c'est à dire une forme permettant l'action des ions fluorure dans la cavité buccale. Une telle forme pour application buccale peut consister en un dentifrice, un spray, une gomme à mâcher, une pastille à sucer, un gel dentaire, un implant buccal tel un patch buccal, notamment un patch mucoadhésif, un bain de bouche. Un dentifrice selon l'invention peut se présenter sous forme pâteuse, liquide, de gel, de poudre ou de mousse.

Ces formes, en elles-mêmes, ainsi que leurs procédés de préparation, sont bien connus de l'homme du métier.
Outre les vecteurs d'ions fluorure, les formes pour application buccale locale mentionnées ci-dessus peuvent comprendre des excipients ou ingrédients conventionnels pour chacune de ces formes.

Les formes pour application buccale de l'invention peuvent contenir des tensio-actifs anioniques, amphotères, zwitterioniques, cationiques ou non-ioniques. Parmi ces derniers, on peut plus particulièrement cités les poly(hydroxypropyl éther), comme le dodécanediol polyglycérolé à 3,5 moles de glycérol, décrits dans le brevet français N°84 08459. Les formes pour application buccale locale selon l'invention peuvent encore comprendre des agents épaississants, des agents de cohésion, des agents édulcorants, humectants ou rafraîchissant, des agents conservateurs, des colorants, des agents arômatisants ou de sapidité, des agents plastifiants, des agents peptisants, des agents antitartre, des cicatrisants, des agents antisaignements, des agents de polissage, des agents anti-plaques comme la chlorhexidine, l'hexétidine, le chlorure de cétylpyridinium ou le delmophinol et/ou des enzymes comme le dextranase, la mutanase, les lysozymes, la lactcferrine ou les peroxydases.

Un médicament selon l'invention permet la prévention ou le traitement adjuvant de maladies associées à Helicobacter pylori, en particulier certaines maladies gastro-intestinales. Parmi ces dernières, on peut plus particulièrement citées les gastrites, les ulcères gastriques ou du duodénum. La demanderesse a pu mettre en évidence que des médicaments comprenant des vecteurs d'ions fluorure pouvaient conduire à l'éradication de Helicobacter pylori. De cette façon, les maladies associées à Helicobacter pylori peuvent être prévenues. Par le terme prévention, il faut comprendre qu'un médicament selon l'invention permet d'éviter l'apparition d'une maladie associée à Helicobacter pylori chez un sujet sain, mais aussi la réapparition ou la récidive d'une telle maladie chez un sujet qui en été guéri.

L'utilisation des vecteurs d'ions fluorure selon l'invention constitue un traitement adjuvant de maladies associées à Helicobacter pylori. On entend par le terme "traitement adjuvant", un traitement qui doit être nécessairement accompagné par un autre traitement mettant en oeuvre d'autres médicaments. En effet, l'utilisation des vecteurs d'ions fluorure selon l'invention, à eux seuls, ne permet pas le traitement de ces maladies. L'association d'un médicament comprenant un vecteur d'ions fluorure à d'autres médicaments est requise en vue d'un traitement efficace desdites maladies associées à Helicobacter pylori.

Ces médicaments sont généralement du type de ceux utilisés de manière classique dans le traitement des maladies gastro-intestinales,notamment celles citées plus haut, en particulier l'ulcère gastrique et l'ulcère duodénal. A ce titre on peut citer des médicaments anti-ulcère comme les inhibiteurs de la pompe à proton, tels l'oméprazole, le pantoprazole, le lanzoprazole, et les antagonistes des récepteurs H₂, tels la ranitidine et ses dérivés, la famotidine et la cimétidine.

Selon un autre aspect de l'invention, celle-ci consiste alors en une composition pharmaceutique comprenant un vecteur d'ions fluorure pharmaceutiquement acceptable et, soit un inhibiteur de la pompe à proton, soit un antagoniste des récepteurs H₂ comme produit de combinaison pour une utilisation simultanée, séparée ou étalée dans le temps pour le traitement ou la prévention de maladies gastro-intestinales associées à Helicobacter pylori, comme l'ulcère gastro-intestinal.

Les médicament comprenant un vecteur d'ions fluorure, outre les médicaments anti-ulcères mentionnés ci-dessus, peuvent encore être associés à au moins un antibiotique actif à l'encontre d'Helicobacter pylori. Un traitement associant un médicament anti-ulcère de type antagoniste des récepteurs H₂ ou inhibiteur de la pompe à proton avec au moins un antibiotique choisi, est connu en soi.

A titre d'antibiotique actif contre Helicobacter pylori, on peut citer la pénicilline et ses dérivés comme l'amoxicilline, la benzylpénicilline, la pipéracilline ; les céphalosporines comme le céfixime et le céfaclor ; les macrolides comme l'érythromycine et la clarithromycine ; la tétracycline et ses dérivés comme mynocycline et la streptomycine ; les aminoglycosides comme la gentamycine et l'amikacine ; les nitro-5-imidazolés comme le métronidazole, ainsi que des associations de ces antibiotiques.

Selon encore un autre aspect de l'invention, celle-ci consiste alors en une composition pharmaceutique comprenant
(i) un vecteur d'ions fluorure pharmaceutiquement acceptable,
(ii) au moins un antibiotique actif contre Helicobacter pylori et (iii) soit un inhibiteur de la pompe à proton, soit un antagoniste des récepteurs H₂, comme produit de combinaison pour une utilisation simultanée, séparée ou étalée dans le temps pour le traitement ou la prévention de maladies gastro-intestinales associées à Helicobacter pylori, comme l'ulcère gastro-intestinal.

L'exemple qui suit a pour but d'illustrer la présente invention.

### Exemple

En vue d'étudier l'efficacité d'un médicament comprenant des vecteurs d'ions fluorure vis à vis d'Helicobacter pylori, on a préparé un dentifrice conforme à l'invention comprenant les composés suivants (% en poids) :
- fluorure de sodium 0,33 %
- monoflurophosphate de sodium 0,76 %
- silice 21,00 %
- carréghénate 1,00 %
- TiO₂ 1,00 %
- phosphates 0,30 %
- benzoate de sodium 4,00 %
- sorbitol 25,00 %
- conservateur 0,10 %
- arômes 1,06 %
- eau qsp 100,00 %

Ce dentifrice comprend 0,25 % en poids d'ions fluorure.

A titre comparatif, on a également préparé un dentifrice non conforme à l'invention (placebo) comprenant les composés mentionnés ci-desssus, à l'exception du fluorure de sodium et du monoflurophosphate de sodium.

On a ensemencé des milieux de culture avec Helicobacter pylori dans des conditions favorisant le développement de colonies de cette bactérie. La souche d'Helicobacter pylori utilisée a été isolée dans le laboratoire d'Epidiémologie des Maladies Infectieuses de l'Université Paul Sabatier de Toulouse, à partir de flore sous-gingivale de patients. Cette souche a été identifiée selon les critèrse de Bergey's Manual, édition 1994.

Puis, on a mis en contact les milieux de culture avec des solutions dans l'eau, soit du dentifrice conforme à l'invention, soit du placebo. Des solutions de ces dentifrices, diluées au 1/2, 1/4, 1/8 et 1/16 ont été testées. On a mis les différentes solutions de dentifrice au contact des colonies d'Helicobacter pylori pendant 5 min., 60 min. et 24 heures.

Les résultats obtenus sont décrits dans le Tableau I (solutions de placebo) et le Tableau II (dentifrice conforme à l'invention) ci-après.

**Tableau I**

| Placebo | Dilution 1/2 | Dilution 1/4 | Dilution 1/8 | Dilution 1/16 |
|---|---|---|---|---|
| 5 min. | 19 (0,9) | 26 (3,5) | 22 (2,6) | 20 (1,6) |
| 60 min. | 17 (0,7) | 14 (3,2) | 15 (1,0) | 19 (4,7) |
| 24 H | 16,2 (7,5) | 12 (1,4) | 50 (1,6) | 51 (5,9) |

**Tableau II**

| Dentifrice selon l'invention | Dilution 1/2 | Dilution 1/4 | Dilution 1/8 | Dilution 1/16 |
|---|---|---|---|---|
| 5 min. | 5,5 (4,0) | 5,5 (2,1) | 6,3 (0,4) | 20 (1,8) |
| 60 min. | 3,2 (0,8) | 5,5 (0,4) | 8,1 (2,2) | 29 (0,9) |
| 24 H | 1,6 (0,6) | 4,5 (1,5) | 9,1 (3,4) | 34 (8,1) |

Les valeurs des tableaux représentent le nombre d'Unités Formant des Colonies (UFC) à multiplier par 10⁵. Chacune de ces valeurs représente une moyenne de 3 tests. La distribution de ces trois valeurs est donnée par la déviation standard figurant entre parenthèse,également à multiplier par 10⁵.

Les résultats obtenus montrent une faible efficacité du placebo,voire une absence d'efficacité, avec des temps de contact de 5 min. ou 60 min.. En revanche, le dentifrice conforme à l'invention réduit le nombre d'UFC de manière significative après seulement 5 min. de contact.
Les déviations standard sont fiables et témoignent d'une bonne reproductibilité des tests.

## Revendications

1. Utilisation d'au moins un vecteur d'ions fluorure pharmaceutiquement acceptable à une teneur en ions fluorure supérieure à 0,15 jusqu'à 1 % en poids pour la préparation d'un médicament destiné à la prévention ou au traitement adjuvant d'une maladie associée à Helicobacter pylori.

2. Utilisation d'au moins un vecteur d'ions fluorure pharmaceutiquement acceptable à une teneur en ions fluorure supérieure à 0,15 jusqu'à 1 % en poids pour la préparation d'un médicament destiné à l'éradication d'Helicobacter pylori dans la cavité buccale.

3. Utilisation selon l'une des revendications 1 et 2, **caractérisée en ce que** le vecteur d'ions fluorure pharmaceutiquement acceptable est choisi dans le groupe constitué par (i) les fluorures d'amine, d'ammonium, de métaux alcalins ou alcalino-terreux, de titane, de zinc, d'aluminium, (ii) les fluorophosphates d'aluminium, de métaux alcalins ou alcalino-terreux, tels que le monofluorophosphate de sodium ou le monofluorophosphate d'aluminium, (iii) le difluorophosphate d'aluminium, (iv) le fluorozirconate de sodium, (v) les fluorosilicates.

4. Utilisation selon l'une des revendications 1 à 3, **caractérisée en ce que** le vecteur d'ions fluorure pharmaceutiquement acceptable est le fluorure de sodium, le monofluorophosphate de sodium ou un mélange de fluorure de sodium et de monofluorophosphate de sodium.

5. Utilisation selon l'une des revendications 1 à 4, **caractérisée en ce que** le médicament se présente sous une forme pour application buccale locale.

6. Utilisation selon la revendication 5, **caractérisée en ce que** la forme pour application buccale est un dentifrice, un spray, une gomme à mâcher, une pastille à sucer, un gel dentaire, un implant buccal tel un patch buccal, un bain de bouche.

7. Utilisation selon l'une des revendications 1 à 6, **caractérisée en ce que** la maladie associée à Helicobacter pylori est l'ulcère gastro-intestinal.

8. Une composition pharmaceutique comprenant un vecteur d'ions fluorure pharmaceutiquement acceptable à une teneur en ions fluorure supérieure à 0,15 jusqu'à 1 % en poids et, soit un inhibiteur de la pompe à proton, soit un antagoniste des récepteurs H₂ comme produit de combinaison pour une utilisation simultanée, séparée ou étalée dans le temps pour le traitement ou la prévention de maladies gastro-intestinales associées à Helicobacter pylori, comme l'ulcère gastro-intestinal.

9. Une composition pharmaceutique comprenant (i) un vecteur d'ions fluorure pharmaceutiquement acceptable à une teneur en ions fluorure supérieure à 0,15 jusqu'à 1 % en poids, (ii) au moins un antibiotique actif contre Helicobacter pylori et (iii) soit un inhibiteur de la pompe à proton, soit un antagoniste des récepteurs H₂, comme produit de combinaison pour une utilisation simultanée, séparée ou étalée dans le temps pour le traitement ou la prévention de maladies gastro-intestinales associées à Helicobacter pylori, comme l'ulcère gastro-intestinal.

## Patentansprüche

1. Verwendung mindestens eines pharmazeutisch annehmbaren Fluoridionen-Vektors mit einem Fluoridionengehalt von mehr als 0.15 bis 1 Gew.-% für die Herstellung eines Arzneimittels zur Vorbeugung oder begleitenden Behandlung einer mit Helicobacter pylori in Beziehung stehenden Erkrankung.

2. Verwendung mindestens eines pharmazeutisch annehmbaren Fluoridionen-Vektors mit einem Fluoridionengehalt von mehr als 0,15 bis 1 Gew.-% für die Herstellung eines Arzneimittels zur Vernichtung von Helicobacter pylori im Mundraum.

3. Verwendung nach einem der Ansprüche 1 und 2, **dadurch gekennzeichnet, daß** der pharmazeutisch annehmbare Fluoridionen-Vektor ausgewählt ist aus der Gruppe, die gebildet wird durch (i) die Fluoride von Aminen, Ammonium, Alkalimetallen, Erdalkalimetallen, Titan, Zink und Aluminium, (ii) Fluorphosphaten von Aluminium, Alkalimetallen oder Erdalkalimetallen, wie Natriummonofluorphosphat oder Aluminiummonofluorphosphat, (iii) Aluminiumdifluorphosphat, (iv) Natriumfluorzirconat und (v) Fluorsilicaten.

4. Verwendung nach einem der Ansprüche 1 bis 3, **dadurch gekennzeichnet, daß** der pharmazeutisch annehmbare Fluoridionen-Vektor Natriumfluorid, Natriummonofluorphosphat oder eine Mischung aus Natriumfluorid und Natriummonofluorphosphat ist.

5. Verwendung nach einem der Ansprüche 1 bis 4, **dadurch gekennzeichnet, daß** das Arzneimittel in einer für die lokale bukkale Verabreichung geeigneten Form vorliegt.

6. Verwendung nach Anspruch 5, **dadurch gekennzeichnet, daß** die Form für die bukkale Verabreichung eine Zahnpasta, ein Sprühpräparat, ein Kaugummi, eine Lutschtablette, ein Zahngel, ein Mundimplantat, wie ein Mundpflaster, oder eine Munddusche ist.

7. Verwendung nach einem der Ansprüche 1 bis 6, **dadurch gekennzeichnet, daß** die mit Helicobacter pylori verknüpfte Krankheit ein Magen-Darm-Geschwür ist.

8. Pharmazeutische Zubereitung umfassend einen pharmazeutisch annehmbaren Fluoridionen-Vektor mit einem Fluoridionengehalt von mehr als 0,15 bis 1 Gew.-% und entweder einen Inhibitor der Protonenpumpe oder einen Antagonisten für H₂-Rezeptoren als Kombinationsprodukt für eine gleichzeitige, getrennte oder zeitlich verschobene Verwendung bei der Behandlung oder der Vorbeugung von mit Helicobacter pylori verknüpften Magen-Darm-Erkrankungen, wie Magen-Darm-Geschwüren.

9. Pharmazeutische Zubereitung umfassend (i) einen pharmazeutisch annehmbaren Fluoridionen-Vektor mit einem Fluoridionengehalt von mehr als 0,15 bis 1 Gew.-%, (ii) mindestens ein gegen Helicobacter pylori wirksames Antibiotikum, und (iii) entweder einen Inhibitor der Protonenpumpe, oder einen Antagonisten für H₂-Rezeptoren, als Kombinationsprodukt zur gleichzeitigen, getrennten oder zeitlich verschobenen Verwendung bei der Behandlung oder der Vorbeugung von mit Helicobacter pylori verknüpften Magen-Darm-Erkrankungen, wie Magen-Darm-Geschwüren.

## Claims

1. Use of at least one pharmaceutically acceptable fluoride ion vector, with a fluoride ion content of greater than 0.15% and up to 1% by weight, for preparing a medicinal product intended for the prevention or the adjuvant treatment of a disease associated with Helicobacter pylori.

2. Use of at least one pharmaceutically acceptable fluoride ion vector, with a fluoride ion content of greater than 0.15% and up to 1% by weight, for preparing a medicinal product intended for the eradication of Helicobacter pylori in the buccal cavity.

3. Use according to either of Claims 1 and 2, **characterized in that** the pharmaceutically acceptable fluoride ion vector is chosen from the group consisting of (i) fluorides of amine, ammonium, alkali metal, alkaline-earth metal, titanium, zinc or aluminium, (ii) fluorophosphates of aluminium, alkali metal or alkaline-earth metal, such as sodium monofluorophosphate or aluminium monofluorophosphate, (iii) aluminium difluorophosphate, (iv) sodium fluorozirconate, and (v) fluorosilicates.

4. Use according to one of Claims 1 to 3, **characterized in that** the pharmaceutically acceptable fluoride ion vector is sodium fluoride, sodium monofluorophosphate or a mixture of sodium fluoride and of sodium monofluorophosphate.

5. Use according to one of Claims 1 to 4, **characterized in that** the medicinal product is provided in a form for local buccal application.

6. Use according to Claim 5, **characterized in that** the form for buccal application is a toothpaste, a spray, a chewing gum, a lozenge to be sucked, a dental gel, a buccal implant such as a buccal patch, or a mouthwash.

7. Use according to one of Claims 1 to 6, **characterized in that** the disease associated with Helicobacter pylori is a gastrointestinal ulcer.

8. Pharmaceutical composition comprising a pharmaceutically acceptable fluoride ion vector, with a fluoride ion content of greater than 0.15% and up to 1% by weight, and either a proton pump inhibitor or an H₂ receptor antagonist, as a combination product for simultaneous, separate or sequential use, for treating or preventing gastrointestinal diseases associated with Helicobacter pylori, such as a gastrointestinal ulcer.

9. Pharmaceutical composition comprising (i) a pharmaceutically acceptable fluoride ion vector, with a fluoride ion content of greater than 0.15% and up to 1% by weight, (ii) at least one antibiotic active against Helicobacter pylori, and (iii) either a proton pump inhibitor or an H₂ receptor antagonist, as a combination product for simultaneous, separate or sequential use, for treating or preventing gastrointestinal diseases associated with Helicobacter pylori, such as a gastrointestinal ulcer.
